# EUROPÄISCHE PATENTSCHRIFT

(11) **EP 0 569 753 B1**
(45) Veröffentlichungstag und Bekanntmachung des Hinweises auf die Patenterteilung: **08.07.1998**
(21) Anmeldenummer: 93106616.1
(22) Anmeldetag: 23.04.1993
(51) Int. Cl.: G01N 33/543, G01N 35/00, C12M 1/20, B01L 3/00

(54) **Verfahren zur Aufnahme, Lagerung und Dispensierung von Scheiben mit daran gebundenen biologisch aktiven Substanzen**
Process for receiving, storing and dispensing plates with bound biologically active substances
Procédé de recevoir, stocker et distribuer des disques avec des substances biologiquement actives

(30) Priorität: 14.05.1992 DE 4215932
(43) Veröffentlichungstag der Anmeldung: 18.11.1993
(73) Patentinhaber: Ringel, Karl-Peter, Prof. Dr., Dublin 14 (IE)
(72) Erfinder: Westhall Speight, John, Dublin 14 (IE)
(74) Vertreter: Patentanwälte Meinke, Dabringhaus und Partner

(56) Entgegenhaltungen:
- WO-A-88/05541
- DE-A- 2 508 820
- GB-A- 2 001 432
- US-A- 3 862 886
- US-A- 4 909 992
- Derwent WPI AN 88-045930 [07] & JP-A-63 003 264 ( TSUKIOKA YASUNOBU ) 08.01.1988

## Beschreibung

Die Erfindung betrifft ein Verfahren zum Bestücken von Probenröhrchen, Mikrotiterplatten, -riegeln oder dergl. mit Scheiben mit daran gebundenen biologisch aktiven Substanzen, vornehmlich Antigene und/oder Antikörper und/oder pharmakologisch wirksame Substanzen in einer automatischen Pipettiereinrichtung mit wenigstens einem rohrförmigen Aufnahmeelement für die Scheiben.

Bei immunologischen Untersuchungen mit Antigenen und/oder Antikörpern, die eine Enzym- und/oder Fluoreszenz- und/oder Radioisotopen- und/oder Chemilumineszenzmarkierung aufweisen, verwendet man gewöhnlich immobilisierte Antigene und/oder Antikörper oder andere biologische Substanzen, die auf einem in ein Probenröhrchen oder dergl. einzuführenden Gegenstand gebunden sind. Insbesondere bei Reihenanalysen ist es üblich, kleine Probenröhrchen zu verwenden, die becherförmig ausgebildet sind und meist zu mehreren in einer Reihe oder einer Fläche miteinander verbunden sind. In diese Probenröhrchen wird bekanntermaßen eine den Boden bedeckende Scheibe aus Papier, Kunststoff oder einem anderen aktivierbaren Träger eingelegt, an welcher das Antigen oder der Antikörper gebunden ist. Nach dem manuellen Einführen der Scheibe in das Probenröhrchen wird die Probenflüssigkeit in das Probenröhrchen eingefüllt, worauf dann in mehreren Stufen nacheinander gewaschen und mit weiteren Reagenzien behandelt wird, um am Ende mit Hilfe der Radio-, Enzym-, Fluoreszenz- oder Chemilumineszenzmarkierung eine qualitative oder quantitative Bestimmung der gesuchten Substanz in der Probenflüssigkeit durchzuführen.

Dabei werden Reagenzien und Flüssigkeiten für Zwischenwaschstufen mit Hilfe einer Pipettiereinrichtung aus dem Probenröhrchen entnommen und durch eine andere Flüssigkeit ersetzt, wozu vollautomatisch Pipettiereinrichtungen bekannt sind, die programmgesteuert die entsprechenden Probenröhrchen füllen bzw. leeren. Durch diese automatischen Pipettiereinrichtungen können derartige Reihentests beschleunigt und Fehlerquellen weitgehend ausgeschlossen werden.

Auch bei derartigen automatischen Pipettiereinrichtungen ist es derzeit jedoch notwendig, zunächst in jedes Probenröhrchen jeweils eine Scheibe mit daran gebundenem Antigen oder Antikörper oder sonstigen pharmazeutischen Aktivstoffen - wie oben ausgeführt - manuell zu positionieren. Ein besonderes Problem stellen dabei Trägerscheiben dar, die nur in Lösungen stabil sind. Durch die manuelle Verteilung und Positionierung dieser Trägerscheiben sind Zeitaufwand, Kosten und Fehlerquellen bei Serienanalysen sehr hoch.

Aus der US-A-3,862,886 ist ein beidseitig verschließbares, rohrförmiges Aufnahmeelement für eine Vorrichtung zum Wachsenlassen von Bakterienkulturen oder dergl. bekannt, wobei in dem rohrförmigen Aufnahmeelement übereinander abwechselnd Scheiben und Abstandhalter angeordnet sind, wobei zentral im Aufnahmeelement ein stabförmiges Element angeordnet ist, auf welches abwechselnd die Scheiben und die Abstandhalter, die jeweils mit einer zentrischen Bohrung ausgestattet sind, aufgesteckt sind.

Aus JP-A-63 003 264 ist eine Pipettiereinrichtung mit einem automatisch steuerbaren verfahrbaren Sauggkopf mit einer Mehrzahl von Saugspitzen bekannt, die Bestandteil einer Waschvorrichtung für Bluttesteinrichtungen ist.

Aufgabe der Erfindung ist die Schaffung einer Lösung, mit der eine Automatisierung der Verteilung und Positionierung derartiger Trägerscheiben ermöglicht wird.

Diese Aufgabe wird mit einem Verfahren der eingangs beschriebenen Art erfindungsgemäß durch die Merkmale des kennzeichnenden Teiles des Patentanspruches 1 gelöst.

Mit diesem Verfahren ist es möglich, eine Vielzahl derartiger Trägerscheiben mit oder ohne Lösungsmittel bereitzuhalten, wobei das rohrförmige Aufnahmeelement bevorzugt als Probenröhrchen ausgebildet ist, so daß es ohne weiteres in Pipettiereinrichtungen eingesetzt werden kann. Durch entsprechende Steuerung der Pipettiereinrichtung kann dann jeweils von der Pipettiereinrichtung eine Trägerscheibe automatisch entnommen und in ein entsprechendes Aufnahmegefäß, vornehmlich ein Probenröhrchen, eingeführt werden, in dem die Trägerscheibe entsprechend von der Pipettiereinrichtung angesaugt wird. Dabei ist durch die alternierende Anordnung von Trägerscheiben und Abstandhaltern gewährleistet, daß jeweils nur eine Trägerscheibe entnommen wird, was insbesondere dann von besonderer Bedeutung ist, wenn in dem rohrförmigen Aufnahmeelement auch noch eine Flüssigkeit enthalten ist, wie z.B. ein Lösungsmittel bzw. -vermittler. In einem solchen Falle ist es nämlich nahezu unmöglich, infolge der Oberflächenspannung der Flüssigkeit reproduzierbar einzelne Scheiben automatisch zu entnehmen, falls die eigens dafür konstruierten Abstandhalter fehlen sollten. Die Pipettiereinrichtung kann mit einer derartigen Einrichtung völlig automatisch betrieben werden, so daß bei großen Analyseserien der Zeitaufwand, die Kosten und die Fehlerquellen stark reduziert werden.

In vorteilhafter Ausgestaltung ist vorgesehen, daß scheibenförmig ausgebildete und mit beidseitigen kegelförmigen Erhebungen versehene Abstandhalter verwendet werden. Die Trägerscheibe liegt dann nur punktuell auf dem Abstandhalter auf, so daß ein einfaches und fehlerfreies Entnehmen durch Absaugen mittels der Pipettiereinrichtung möglich ist. Besteht der Abstandhalter aus chemisch inertem Material, so ist gewährleistet, daß dieser auch bei Einsatz von Lösungsmitteln bzw. -vermittlern oder durch den Kontakt mit den Trägerscheiben beständig bleibt und somit auch keine verändernden Einflüsse auf die an der Scheibe gebundenen Komponenten erfolgt.

Die Erfindung ist nachstehend anhand der Zeichnung beispielsweise näher erläutert. Diese zeigt in:
- Fig. 1: teilweise im Schnitt eine Einrichtung zur Aufnahme und Lagerung von Trägerscheiben,
- Fig. 2: eine Aufsicht auf einen Abstandhalter und in
- Fig. 3: in stark vereinfachter Darstellung in Seitenansicht eine Pipettiereinrichtung.

Eine Einrichtung zur Aufnahme und Lagerung von Scheiben mit daran gebundenem Antigen oder Antikörper bzw. anderen chemisch gebundenen pharmazeutischen oder biologischen Aktivstoffen - wie zuvor ausgeführt - für Probenröhrchen o.ä. ist in der Zeichnung allgemein mit 1 bezeichnet. Die Einrichtung weist ein rohrförmiges Aufnahmeelement 2 auf, das bevorzugt die Form eines derartigen Probenröhrchens hat und verschließbar ist, was in der Zeichnung nicht im einzelnen dargestellt ist. Das rohrförmige Aufnahmeelement 2 besteht bevorzugt aus transparentem Kunststoff.

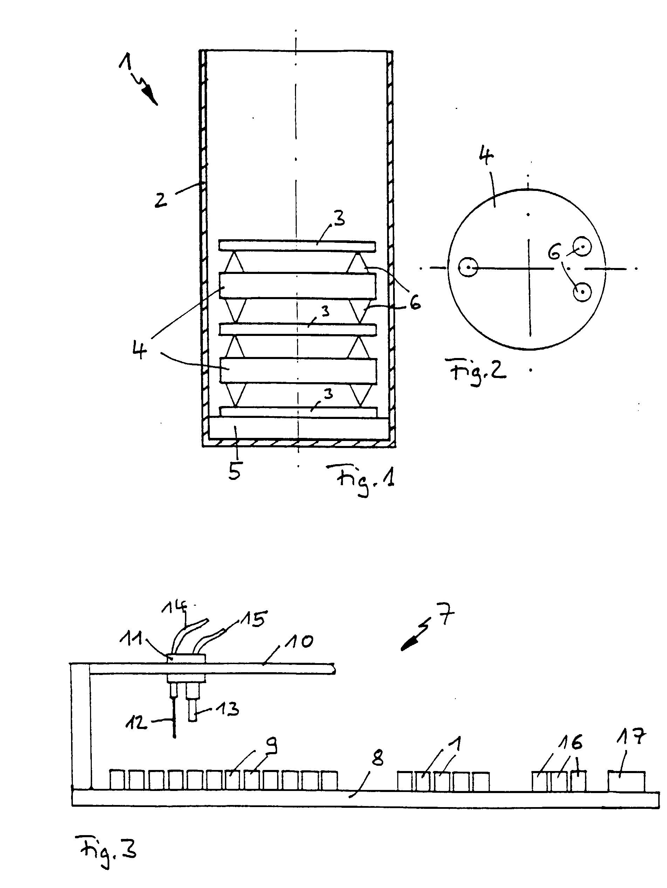

Im rohrförmigen Aufnahmeelement 2 sind alternierend Trägerscheiben 3 und Abstandhalter 4 angeordnet, wobei die unterste Trägerscheibe 3 bevorzugt auf einem Schwamm 5 an der Bodenfläche des rohrförmigen Aufnahmeelementes 2 angeordnet ist. Die Abstandhalter 4 sind ebenfalls scheibenförmig ausgebildet (Fig. 2) und mit beidseitigen kegelförmigen Erhebungen 6 versehen. Dabei bestehen die Abstandhalter bevorzugt aus chemisch inertem Material.

Werden Trägerscheiben 3 eingesetzt, die nur in Lösungen stabil sind, ist das rohrförmige Aufnahmeelement 2 bevorzugt mit Lösungsmittel aufgefüllt. Durch die alternierende Anordnung von Abstandhaltern 4 und Trägerscheiben 3 sowie durch die Ausbildung der Abstandhalter 4 ist gewährleistet, daß die Trägerscheiben 3 getrennt voneinander angeordnet sind und insbesondere auch nicht an den Abstandhaltern 4 anhaften können, so daß zuverlässig sichergestellt ist, daß durch Absaugen mittels einer Pipettiereinrichtung jeweils nur eine Trägerscheibe 3 aus der Einrichtung 1 entnommen wird.

Von einer Pipettiereinrichtung 7 sind in Fig. 3 nur die für die Erfindung wesentlichen Teile dargestellt, die übrigen Bestandteile der Einrichtung sind bekannter Stand der Technik, so daß hierauf nicht im einzelnen eingegangen wird.

Die Pipettiereinrichtung 7 weist zunächst eine Aufnahmefläche 8 für Probenröhrchen o.ä. auf, die bevorzugt in Mikrotiterplatten bzw. -riegelanordnung miteinander verbunden sind. Diese Probenröhrchen sind mit 9 bezeichnet. Darüber hinaus weist die Pipettiereinrichtung 7 einen Führungsrahmen 10 auf, in dem vollautomatisch gesteuert ein Saugkopf 11 verfahrbar angeordnet ist, wobei der Saugkopf 11 in alle drei Raumrichtungen bewegbar ist. Der Saugkopf 11 ist mit zwei Saugspitzen 12, 13 ausgerüstet, denen entsprechende Saugleitungen 14, 15 zugeordnet sind. Diese Saugspitzen 12, 13 sind, wie dargestellt, nebeneinander angeordnet, sie können aber auch revolverkopfartig angeordnet sein. Mit der dargestellten Anordnung kann die Saugspitze 12 bzw. 13 automatisch in den Saugkopf 11 verfahren werden, derart, daß jeweils nur eine Saugspitze 12 oder 13 zum Einsatz kommt.

Auf der Aufnahmefläche 8 sind darüber hinaus erfindungsgemäße Einrichtungen 1 angeordnet, die bevorzugt dieselbe Größe aufweisen, wie die Probenröhrchen 9. Ferner sind darüber hinaus auch noch Behälter mit der zu untersuchenden Probe, z.B. Röhrchen 16, vorgesehen sowie ein Sammelgefäß 17, das zum Sammeln der Abstandhalter dient.

Der Verfahrensablauf zur Bestückung der Probenröhrchen 9 mit Trägerscheiben 4 ist der folgende:

Die Pipettiereinrichtung 7 entnimmt vollautomatisch rechnergesteuert aus den jeweiligen Aufnahmeelementen 1 jeweils eine Trägerscheibe 4 durch Ansaugen und legt diese anschließend in ein Probenröhrchen 9 o.ä. ab. Dabei wird zu diesem Transportvorgang die Dosierspitze 13 eingesetzt, die einen größeren Saugquerschnitt als die Dosierspitze 12 aufweist, derart, daß eine zuverlässige Entnahme der Trägerscheiben gewährleistet ist.

Anschließend wird aus dem betreffenden rohrförmigen Aufnahmeelement 1 der darunter angeordnete, dann obenaufliegende Abstandhalter 3 durch Absaugen entnommen und anschließend abgelegt, und zwar bevorzugt in das Sammelgefäß 17. Wenn die Trägerscheibe 3 aus partiell luftdurchlässigem Material gefertigt ist, kann gleichzeitig durch die unterschiedlichen Druckverhältnisse beim Absaugen in der Dosierspitze 11 überwacht werden, ob jeweils, wie gewünscht, eine Trägerscheibe 3 oder ein Abstandhalter 4 entnommen wird.

Nach der vollständigen Bestückung sämtlicher Probenröhrchen 9 mit Trägerscheiben 3 kann der eigentliche Arbeitsablauf in der Pipettiereinrichtung erfolgen, was für sich betrachtet bekannt ist, so daß hierauf nicht im einzelnen eingegangen wird. Dazu wird dann automatisch die Dosierspitze 12 verwandt, die Dosierspitze 13 ist dann außer Funktion.

Der Abstandhalter 4 der Einrichtung 1 kann anders ausgebildet sein, auch die Pipettiereinrichtung 7 kann eine andere Ausgestaltung aufweisen, wobei allerdings eine zuverlässige, fehlerfreie automatische Entnahme der Trägerscheiben 3 aus den Aufnahmeelementen 1 gewährleistet sein muß.

## Patentansprüche

1. Verfahren zum Bestücken von Probenröhrchen, Mikrotiterplatten oder -riegeln bzw. anderen Gefäßen mit Scheiben mit daran gebundenen biologisch aktiven Substanzen in einer automatischen Pipettiereinrichtung mit wenigstens einem rohrförmigen Aufnahmeelement für die Scheiben,
dadurch gekennzeichnet,
daß vollautomatisch von der Pipettiereinrichtung aus dem rohrförmigen Aufnahmeelement, in dem alternierend übereinander eine Mehrzahl von Scheiben und Abstandhaltern angeordnet sind, jeweils eine Scheibe durch Ansaugen entnommen und anschließend in ein Probenröhrchen abgelegt wird, und daß dann der im rohrförmigen Aufnahmeelement obenliegende Abstandhalter durch Ansaugen von der Pipettiereinrichtung entnommen und abgelegt wird.

2. Verfahren nach Anspruch 1,
dadurch gekennzeichnet,
daß der im rohrförmigen Aufnahmeelement obenliegende Abstandhalter durch Ansaugen entnommen und separat insbesondere in einem Sammelgefäß abgelegt wird.

3. Verfahren nach Anspruch 1 oder 2,
dadurch gekennzeichnet,
daß scheibenförmig ausgebildete und mit beidseitigen kegelförmigen Erhebungen versehene Abstandhalter verwendet werden.

## Claims

1. A method of equipping test tubes, microtitre plates or bars or other vessels with discs with biologically active substances bonded thereto in an automatic pipetting device with at least one tubular receiving element for the discs, characterised in that in a fully automatic procedure a respective disc is removed by suction by the pipetting device from the tubular receiving element in which a plurality of discs and spacers are arranged alternately in mutually superposed relationship, and then deposited in a test tube, and that then the spacer which is at the top in the tubular receiving element is removed by suction by the pipetting device and deposited.

2. A method according to claim 1 characterised in that the spacer which is at the top in the tubular receiving element is removed by suction and deposited separately, in particular in a collecting vessel.

3. A method according to claim 1 or claim 2 characterised in that spacers which are of a disc-like configuration and which are provided with conical raised portions on both sides are used.

## Revendications

1. Procédé pour équiper des tubes à essais, des plaques ou des barrettes de microtitrage ou d'autres récipients comportant des disques, auxquels sont fixées des substances actives du point de vue biologique, dans un dispositif automatique de prélèvement à la pipette, avec au moins un élément tubulaire de réception pour les disques,
caractérisé en ce
que respectivement un disque est prélevé par aspiration, d'une manière entièrement automatique, par le dispositif de prélèvement à la pipette à partir d'un élément tubulaire de réception, dans lequel sont disposés d'une manière alternée et superposée une multiplicité de disques et d'entretoises, puis est déposé dans un tube à essais, et qu'ensuite l'entretoise supérieure dans l'élément tubulaire de réception est retiré par aspiration par le dispositif de prélèvement à la pipette et est rangé.

2. Procédé selon la revendication 1, caractérisé en ce
que l'entretoise supérieure dans l'élément tubulaire de réception est retirée par aspiration et est déposée séparément notamment dans un récipient de collecte.

3. Procédé selon la revendication 1 ou 2, caractérisé en ce
qu'on utilise des entretoises agencées en forme de disques et qui sont pourvues de bossages de forme conique sur leurs deux faces.
